# EUROPEAN PATENT APPLICATION

(11) **EP 3 889 597 A1**
(43) Date of publication of application: **06.10.2021**
(21) Application number: 19889769.6
(22) Date of filing: 13.11.2019
(51) Int. Cl.: G01N 29/06, G01N 29/07, G01N 29/11, G01N 29/14, G01N 29/34

(54) **IN-LINE SYSTEM FOR DETECTING THE QUALITY OF FRUIT VIA ULTRASOUND**

(30) Priority: 30.11.2018 CL 20183416
(71) Applicant: Universidad De Concepcion, Concepción (CL)
(72) Inventor: RADRIGAN EWOLDT, Rudi, Concepción (CL); ROJAS CABALÍN, Sixto, Concepción (CL)
(74) Representative: Clarke Modet & Co.
(86) International application number: PCT/CL2019/050114
(87) International publication number: WO 2020/107133

(57) **Abstract**

In-line ultrasonic fruit quality detection system comprising: a support arch located above the process line perpendicularly, on which 2 transducers are fixed; a detection unit coupled to the support arch comprising a piezoelectric transmitter and an oscillation band, directed towards the center of the process line; a control unit comprising an analog wave receiving module; an A/D converter; and a microcontroller; a synchronization rotary encoder located under the process line, connected to the control unit of the detection unit; wherein said encoder is coupled to a discard unit for removing from the process line of the fruits that were detected with defect; and a wave generator connected to the control unit. Use of the detection system.

## Description

### Field of the invention

The technology is directed to the fruit-growing field, more particularly, it corresponds to a system for in-line ultrasonic detection of fruit quality.

### Background of the invention

The export of kiwifruits in Chile has reached values in the order of 1.3 million tons of harvested units, with an increase in production of 38% between 2001 and 2010. This ovoid-shaped fruit is covered by a thin brown skin, a green pulp and in its center is the columella, of white-cream color, with elongated shape in the same direction of the length of the fruit.

The main problem for exporting kiwifruits is that they can have a hard columella, which occurs when the center of the fruit does not ripen at the same time as the pulp. Generally, this phenomenon manifests itself between 16 and 24 weeks after harvest and storage. This disorder can occur for a variety of reasons, primarily exposure of harvested kiwifruits to carbon dioxide levels above 8% during the storage period (Postharvest, UC Davis, USA). Another defect is due to fruits are harvested before physiological ripening, which generates a desynchronization of columella and pulp ripening.

While there are methods for determining texture in fruits that can be applied to kiwifruits, they only measure the firmness of the pulp and not the hardness of the columella because it is located in the center of the fruit. In addition, most are destructive in nature, so they are time consuming and not applicable for in-line inspections. An example of such devices is the penetrometer that measures the surface layer of the fruit.

On the other hand, non-destructive systems based on near infrared spectroscopy (NIR) and magnetic resonance have been developed to measure the texture property and firmness of the fruit, which are based on obtaining values from the emission and reception of waves. However, these methods only give a single value for the fruit, and are developed focusing on the physical properties of the pulp and not on the characteristics of the fruit core. In general, most of the studies performed on non-destructive methods only study correlations between pulp firmness measurements by penetrometers and the values delivered by the system, and therefore do not provide information on the different sections of a fruit. In addition, most firmness detection systems allow the measurement of only some specimens as a representative sample of the harvest, which is a limitation due to the fact that fruits may present different stages of physiological ripening at the time of harvest. In addition, it has been reported that there may be fruits of different ripening that come from the same plant, so that the sampling and determination of fruit firmness entail an important percentage of error.

Some documents related to texture determination methods in fruits are listed below:
1. Automated detection of softening and hard columella in kiwifruits during postharvest using X-ray testing", Mondragon et al. (2011). It discloses the determination of hard columella in kiwifruits using X-rays. This measurement is performed by cutting them in half and taking the image of the cross section, wherein the images are processed according to color through software.
2. "Multi-sensor data fusion in the nondestructive measurement of kiwifruit texture", Pourkhak et al, 2017. It discloses different methods for measuring kiwifruit pulp firmness such as drop impact, forced impact and acoustic impulse response. In addition, a correlation of these parameters is performed and the modulus of elasticity and Magness-Taylor firmness value are determined. Finally, it is concluded that it is necessary to use combined methods to have an accurate characterization of kiwifruit texture.
3. Patent US 9,442,055 (Benedetti et al.) entitled: "Process and apparatus for the measurement of the hardness and for the selection of agricultural products". It covers a fruit texture and firmness sensor consisting of a transmitter and an ultrasound receiver coupled to handles adapted to hold the fruit, arranged at an angle of 180°, which are connected to a wave generator and a processor that generates the required information.
4. "Assessing plum fruit quality attributes with an ultrasonic method", Amos Mizrach (2003). It discloses a system for detecting the texture of peach pulp, wherein the fruit is divided in two and the pit is removed. This system allows monitoring of ripeness during storage time, using a transducer and a piezoelectric receiver, a wave generator, a measurement base and an electrical converter.

Based on this background, there is still a need to find non-destructive alternatives to determine the quality of kiwifruit directly in the processing lines.

### Brief description of the figures

Figure 1: General scheme of the system for in-line detection of hard columella in kiwifruits by ultrasound.
Figure 2: Images of the ultrasonic detection system, wherein (a) corresponds to a view of the transducers, (b) to a top view of the system, (c) to a front view of the system and (d) to the user interface.
Figure 3: Graph of firmness variation over harvest time for columella (A) and pulp (B) of kiwifruits.
Figure 4: Ultrasound signal treatment graphs, where (a) corresponds to the pure sensor signal, (b) to the filtered signal and cutoff threshold determination, and (c) to the comparison signal, for sections: (1) peel, (2) pulp, (3) columella, (4) pulp, and (5) peel.

### Detailed description of the invention

The present technology corresponds to a system for in-line ultrasonic detection of kiwifruit quality, more particularly, to determine the state of the columella by ultrasound.

This system is installed directly on the processing line and allows to discriminate the ultrasound signals received from the different layers of the fruit, and to perform the corresponding determination in the center of the fruit. Advantageously, the arrangement of the system produces a narrow detection range that allows determining the firmness properties of one specimen at a time, and ensures detection of the columella section, regardless of the position of the kiwifruit in the processing line.

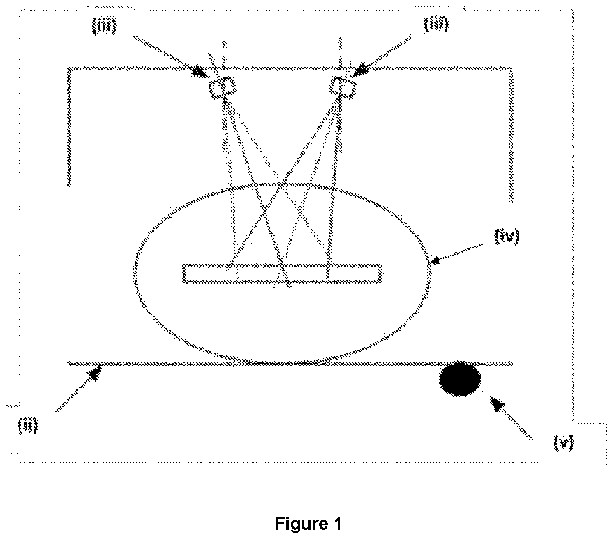

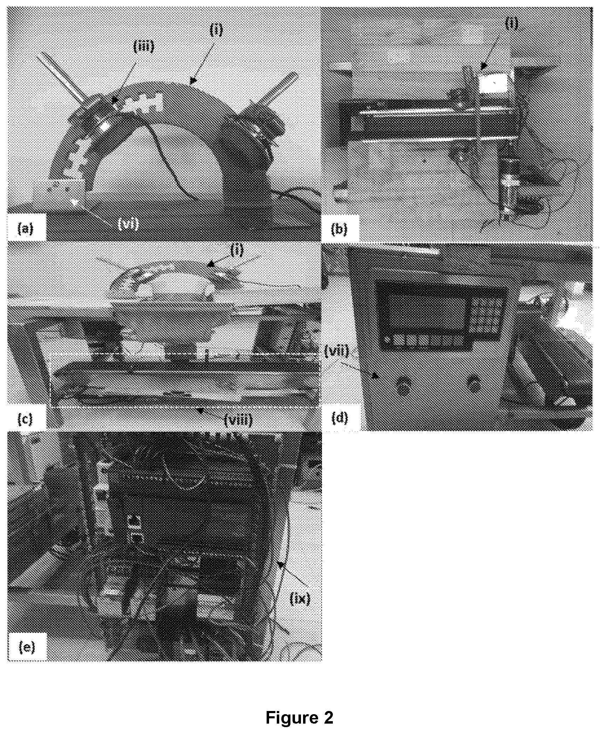

The in-line detection system comprises at least the following components, for which reference is made to Figures 1 and 2:
a. a support arch (/) located on the process line (//) perpendicularly, on which at least 2 transducers are fixed at a height between 5 - 8 cm in relation to the base of the process line (//);
b. at least one sensing unit (///) coupled to the arc support (/) comprising a piezoelectric type transmitter operating between 90 - 1 10 kHz and at an oscillation band between 4 - 7 kHz; and at least one piezoelectric type receiver operating between 90 - 1 10 kHz, directed towards the center of the process line and at a distance from the kiwifruit between 1,5 - 3 cm, with an angle between 20° - 40° to the base of the processing line. Depending on the number of rows of fruit in the processing line, the system can have more than one detection unit (///) to measure all the fruit;
c. a control unit (w7) comprising an analog wave receiving module, which filters and conditions the signal; an A/D converter; and a microcontroller that configures the pulse emission and determines the portion of the wave containing kiwifruit heart hardness information (/V);
d. a synchronizing rotary encoder (v) located below the process line (//), connected to the control unit of the detection unit (///) to coordinate the emission of ultrasonic waves with the passage of the fruit. This encoder (v) can be coupled to a discard unit (w77), comprising a fruit conveyor belt located at a distance of at least 10 cm downstream of the detection chamber, a bypass actuator for belt change, and a PLC that coordinates with the rotary encoder (v) for the removal from the processing line of the fruits that were detected with defect (hard columella); and
e. a wave generator (ix) connected to the control unit (v/7) which produces the wave pulse and delivers it to the transmitter connected to the control unit (v/7).

The detection unit (///) and the discard unit {vi 7/), can be assembled on the process line without altering its structure, allowing the device to be adapted to different structures and processing plants.

Optionally, the support arch (/) may contain a metallic protection {vi) consisting of lateral metallic fins of at least 10 cm, which allow the absorption of the deflected ultrasonic waves.

This system allows the detection of hard columella at a rate of at least 60 fruits per minute, with an accuracy of at least 93%. Advantageously, this system is non-destructive, since it performs the measurement and if the fruit does not present problems it can continue with the packaging process, and if not, it can be separated and sent to other processes to be used, for example, to make juices or jams.

### Application examples

### Example 1. Correlation between Amplitude and Firmness of the columella.

This trial evaluated the columella and pulp firmness of different kiwifruit over a period of time from 0 to 12 days after fruit harvest.

A manual penetrometer Inderst model FT327 was used as a control, where 30 fruits per day were evaluated, from which one end was cut to extract the columella and pulp from each one, to finally measure firmness.

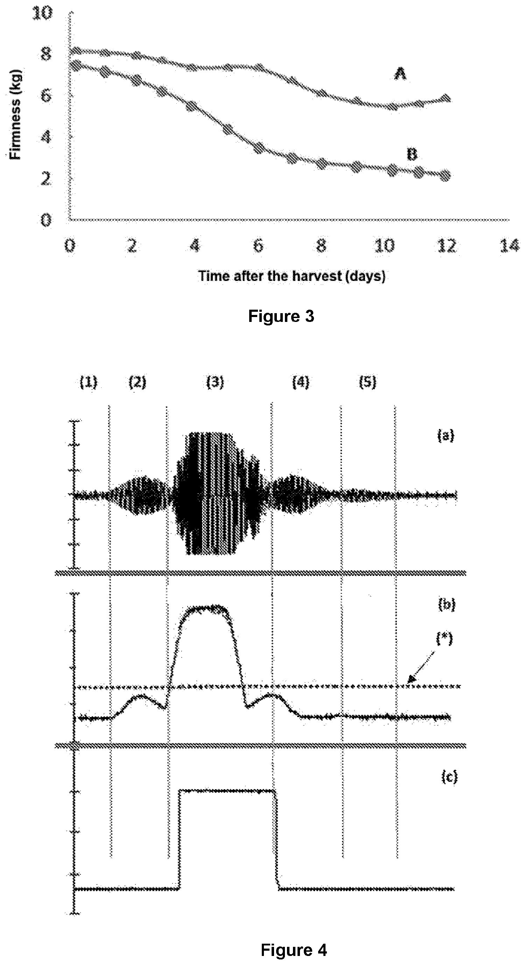

Figure 3 shows a graph showing a firmness curve v/s days after harvest for prematurely harvested kiwifruit, wherein (A) corresponds to the columella curve and (B) to the pulp curve. From this figure it is observed that the firmness curve of columella and pulp are desynchronized, and these are statistically perceived after 3 days, where the differences are significant as a function of firmness trend. Thus, the difference between the firmness values of both the columella and pulp at 3 days is small, which is why the accuracy of the response of the sensors must be high, in order to early determine the hardness of the columella, since it is not possible to wait 12 days to send the fruit to the destination market, which incurs storage costs and loss of time in transport, in addition to a reduction in the days of shelf life due to overripening.

### Example 2. Evaluation of the in-line detection system.

In order to evaluate the ability of the in-line system to detect the different parts of the fruit, 15 kiwifruits were analyzed. This considering, that at first sight there are no differences in the fruit, but only a change in the tonality and hardness detected only when consumed.

The response generated for each part of the fruit was obtained using a GWinstek oscilloscope mod GDS-1022. Figure 4 shows the different sections of the kiwifruit: (1) peel; (2) pulp; (3) columella; (4) pulp and (5) peel, which were evaluated for a single response of the envelope (a), wherein the differences in amplitudes between the different parts of the kiwifruit can be seen. Subsequently, we proceeded to generate an envelope filter and determine a threshold to establish a limit, where above this the columella is hard and below it is soft (4b); from this analysis we obtained a comparator (4c), where if the value of the comparator is above the threshold (*) it indicates a signal of selector or discard of the fruit indicating that it does not meet the threshold criterion.

This analysis can be applied to any fruit to determine its different component parts.

### Example 3: Validation of the detection system in processing lines.

A small kiwifruit sorting line was set up to simulate real process conditions. Fruits were processed on this line at different speeds: 120 fruits/minute, 80 fruits/minute and 60 fruits/minute, which were numbered to identify the optimum processing speed.

After passing the fruits through the detection system, the kiwifruits were cut and their firmness was measured with a manual penetrometer (Indert model Ft327). This allowed determining that the system was able to identify the hard columella in the kiwifruits, reaching a higher effectiveness for a speed of 60 fruits/minute. A total of 465 fruits were evaluated at this speed, obtaining an effectiveness of 93%. For a speed of 120 fruits/minute, 215 fruits were evaluated with an effectiveness of 43% and for a speed of 80 fruits/minute, 320 fruits were analyzed with an effectiveness of 64%.

This trial made it possible to establish the effectiveness of the measurement of the in-line detection system, which facilitates verification directly on the process line for all fruits without the need for intervention.

## Claims

1. A system for in-line ultrasonic detection of fruit quality **CHARACTERIZED in that** comprises at least the following components:
a. a support arch (/) located on the process line (//) perpendicularly, on which at least 2 transducers are fixed at a height of between 5 - 8 cm in relation to the base of the process line (//);
b. at least one detection unit (iii) coupled to the support arc (/) comprising a piezoelectric type transmitter operating between 90 -1 10 kHz and at an oscillation band of between 4 - 7 kHz; and at least one receiver operating between 90 - 1 10 kHz, directed towards the center of the process line and at a distance from the fruit between 1,5 - 3 cm, with an angle of between 20° - 40° with respect to the base of the process line;
c. a control unit (vii) comprising an analog wave receiving module, which filters and conditions the signal; an A/D converter; and a microcontroller that configures the pulse emission and determines the portion of the wave containing the fruit core hardness information(/V);
d. a synchronizing rotary encoder (v) located below the process line (//), connected to the control unit of the detection unit (iii) to coordinate the emission of ultrasonic waves with the passage of the fruit; wherein said encoder (v) is coupled to a discard unit (viii), comprising a fruit conveyor belt located at a distance of at least 10 cm downstream of the detection chamber, a bypass actuator for belt change, and a PLC that coordinates with the rotary encoder (v) for the removal from the processing line of the fruits that were detected with defect; and
e. a wave generator (ix) connected to the control unit (vii) that produces the wave pulse and delivers it to the transmitter connected to the control unit (vii).

2. A system for in-line ultrasonic detection of fruit quality according to claim 1, **CHARACTERIZED in that** the receiver is a piezoelectric type.

3. A system for in-line ultrasonic detection of fruit quality according to claim 1, **CHARACTERIZED in that**, optionally, the support arch (/) has a metallic protection (vi) comprising lateral metallic fins of at least 10 cm to allow the absorption of the deflected ultrasonic waves.

4. Use of the ultrasound detection system, **CHARACTERIZED in that** it functions to determine the hard columella in kiwifruit.
